(19) Europäisches Patentamt
European Patent Office
Office européen des brevets

(11) Publication number: **0 376 870 A1**

(12) # EUROPEAN PATENT APPLICATION

(21) Application number: **89500046.1**

(22) Date of filing: **18.04.89**

(51) Int. Cl.5: **C07F 9/09, C07D 215/56, C07D 471/04, C07D 498/06, C07F 7/18, C07C 229/30**

Claims for the following Contracting State: ES.

(30) Priority: **30.12.88 ES 8804024**

(43) Date of publication of application: **04.07.90 Bulletin 90/27**

(84) Designated Contracting States: **AT CH DE ES FR GB IT LI NL SE**

(71) Applicant: **CENTRO MARGA PARA LA INVESTIGACION S.A.**
**Muntaner 212, 5o-510**
**E-08036 Barcelona(ES)**

(72) Inventor: **Palomo-Nicolau, Francisco Eugenio**
**Dos de Mayo 34, 2o-1a**
**E-08190 Sant Cugat del Vallés Barcelona(ES)**
Inventor: **Cabre-Castellvi, Francisco**
**Casp 114, esc. B, 3o-1a**
**E-08010 Barcelona(ES)**
Inventor: **Cabre-Castellvi, Juan**
**Casp 114, esc. B, 3o-1a**
**E-08010 Barcelona(ES)**
Inventor: **Ballester-Rodes, Montserratt**
**Marqués de Santa 14, 2a**
**E-08023 Barcelona(ES)**
Inventor: **Palomo-Coll, Antonio Luis**
**Escuelas Pias 18, 5o-1a**
**E-08017 Barcelona(ES)**

(74) Representative: **Ballester Rodés, Albert**
**Muntaner 212, 5o- 508**
**E-08036 Barcelona(ES)**

(54) **A new organosilylpolyphosphoric reagent, its preparation and application to the process of synthesis of 3-carboxyquinolones or azaquinolones and their salts.**

(57) A process for the preparation of new organosilylpolyphosphates from the reaction of phosphorus pentoxide with a siloxane or alkyloxysilane to obtain, in solution, reagents the composition of which is $[P_2O_5]_N$[SILANE], where $1 < N < 10$ where N is a function of temperature, reaction time and solvent, being applied to the modulation of cyclization reaction of N-susbtituted aminomethylenemalonates leading to quinolones or azaquinolones.

The N-substituted aminomethylenemalonates are prepared by the reaction of anilines with dialkyl trimethylsilyloxymethylene-malonates.

EP 0 376 870 A1

# A new organosilylpolyphosphoric reagent, its preparation and application to the process of synthesis of 3-carboxyquinolones, or azaquinolones and their salts

The quinolones provided with antibacterial properties include a group defined as derivatives of 1-substituted-1,4-dihydro-4-oxo-3-piridincarboxylic acids. They may be provided with a condensed ring, such as in known cyprofloxacine, or with a system of condensed rings as in the ofloxazine and the flumequine. They constitute a set of compounds of interest in human medicine, veterinary and agriculture. Some are outstanding as fungicides, viricides, coccidiostatics while other are significant because of their anti-ulcer, anti-allergic, anxiolytic, anti-inflammatory and analgesic activity.

Presently, the preparation of these compounds is effected by cyclization processes which consist alternatively through two different routes: (1) chlorine-amine (enamine) exchange reaction, and (2) cyclodealkylation or cycloacylation, with alcohol elimination.

Route (1) has been described in Patents DE 3441788 A1 and ES 548843, and by Grohe and Helmutt (Liebigs Ann. Chem. 1987, 29-37; Bayer A.G.) which develop the cyclization of a class of ethyl 2-(2-chlorobenzoyl)-3-cyclopropylaminoacrylate, substituted at the 3,4,5 positions, with potassium carbonate in dimethylformamide, at reflux temperature (140-150 °C), during 2 hours. When starting from the substituted acid it represents a cumbersome sequence involving at least five stages.

Route (2) is a cycloacylation reaction of an N-substituted diethyl aminomethylenemalonate resulting from the reaction of an aniline, aminopiridine, aminopiridacine or tetrahydroquinoline with diethyl ethoxymethylenemalonate. The methods differ in their cyclization agents:

1. Refluxing in diphenylether or in Dowtherm A, at 200-265° temperature range. Among numerous publication, the procedure is described by Price and Roberts (J.Am.Chem. Soc., 68, 1204, 1946), Riegel at al. (J.Am.Chem.Soc., 68, 1264, 1946). Stanley (J.Am.Chem.Soc. 68, 1277, 1946), Lappin (J.Chem.Educ., 28, 126, 1951), and more recently, Krishnan and Lang (J.Pharm.Sci., 75(12), 1185, 1986), Sanchez and Rogowski (J.Heterocyclic Chem., 24, 215, 1987), and Patents US 3,287,458 (Warner-Lambert Pharm. Co.), JP 80,33453 (Dainippon Pharm. Co. Ltd.), JP 81,128764 (Kyorin Pharm. Co. Ltd.), ES 540226 (Instituto de Investigación y Desarrollo Quimico y Biológico S.A.) and JP 74 88,882, JP 75 49,286 (Yamanouchi Pharm. Co. Ltd.)
2. In paraffin at 260-290 °C. Duffin and Kendall (J.Chem.Soc., 893-895, 1948).
3. Thermal cyclization. Schroetter et al. (J.Prakt. Chem., 320(6), 937-944, 1978).
4. Heating in biphenyl. Suschitzky et al. (J.Chem. Soc., Perkin Trans., 1(23), 2409-2413, 1975).
5. With phosphorus pentoxide, at reflux temperature. Hungarian Patent 11,065 (Chinoin Giog.; Chem.Abstracts 85: 192584t), Nakagome et al. (J.Heter.Chem. 8, 357, 1971), Markees et al. (Helv.Chim.Acta, 55(4), 1319, 1972 and J.Med.Chem., 17, 137-139, 1974).
6. Polyphosphoric acid (PPA), at 130-220 °C. Patents US 4,472,405, 4,585,868, and GB 1,417,129 (Riker Laboratories Inc.), Ger.Offen. 3,007,006, BE 891537 (Otsuka Pharmaceutical Co. Ltd.), EP Appl. 79,162, US Appl. 318930 (Riker Laboratories Inc.), JP 61 143,364 (86 143,364), JP 62 26,272 (87 26,272) (Kyroin Pharmaceutical Co. Ltd.).
7. Ethyl polyphosphate (PPE) at 145 °C. EP 47005 (Daiichi Seiyaku) and Nakagome et al. (J.Heterocycl. Chem., 8, 357, 1971).

Significant difficulties, inconveniences and limitations have been described which are associated to the method of preparation of the N-substituted diethyl aminomethylenemalonate, to the nature of the amines, and to the cyclization reaction. The first interpretations have been given by Duffin and Kendall (loc. cit.). According to them, impurities in the starting materials, particularly those in the diethyl etoxymethylenemalonate (EMME) and those in the diethyl anilinomethylenemalonate (AMME) have a negative influence. Also, byproducts resulting from the excess of primary amine, which cause the cyclization to take place causing darkening, hampering purification and leading to low yields. Raychaudhuri et al. (J. Indian Chem. Soc., 47(1), 25-32, 1970) study the formation of by-products derived from thermolysis of the ethyl 7-chloro-1,4-dihydro-4-oxo-3-quinolinecarboxylate in boiling diphenylether, being 7-chloro-4-ethoxyquinoline, 7-chloro-1-ethyl-4(1H)quinolone with the formation of carbon dioxide. Those authors assign an important role to the purity of the EMME employed in the cyclization of the 3-chloro-AMME. According to Lappim (J.Am.Chem.Soc., 70, 3348-3350, 1948) when temperature is raised (above 110 °C) the yield of the reaction of 2-aminopiridines with EMME deminishes and the formation of deeply colored products increases.

Nakagome et al. (loc. cit.) have reported that the N-ethyl-AMME and the 3,4-dimethoxy-N-ethyl-AMME do not cyclize in Dowtherm. Nevertheless, in PPA they reached yields of 76 and 79% (crude product), respectively, and pointed out that when the cyclization temperature is increased (250-255 °C) the N-ethyl-AMME causes ester hydrolysis and a decrease in yield, the corresponding acid acid being obtained (48%).

They also observe that replacement of PPA by PPE or its trifluoroborate the yields become 72% (ester) and 74% (acid). However, with acetic anhydr-ide/sulfuric acid, the yield of 1-ethyl-1,4-dihydro-4-oxo-3-quinolinecarboxylic acid deminishes (54%). The most dramatic effect takes place with phosphorus pentoxide in boiling benzene (ester, 17%; liberated N-ethylaniline, 28%). Zinc chloride in acetic anhydride/acetic acid, or aluminum chloride in melt are virtually ineffectual.

Markees et al. (loc. cit., Helv.Chim.Acta) corroborate those cyclization deficiencies in boiling diphenylether and that the condensation of secondary amines with EMME requires higher temperatures and extended reaction times than that with primary amines (Markees et. al., J.Med.Chem., loc. cit). Those authors prescribe the use of PPA instead of phosphorus pentoxide for the cyclization of the lesser active AMMEs.

Brown et Dewar (J.Org.Chem., 43(7), 1331-1337, 1978) ascertain that the crucial step of their synthesis is the cyclization of diethyl 4-methoxy-3-piridineaminomethylenemalonate in diphenylether or Dowtherm A. Those authors find also a critical yield dependency upon reaction time: 3 min, 6%; 17 min, 38%, 25 min., 65-75%; higher reaction times causes the formation of other products, the yield being consequently decreased.

Leir (US 4,565,872, Riker Laboratories Inc.) describes the partial hydrolysis of the cyano group in the cyclization of diethyl 2-N-(5-cyano-6-fluorotetrahydroquinaldinyl)methylenemalonate with PPA.

In some compounds provided with functional substituents, such as the 6-fluoro-7-chloro-1-ethylquinolone-3-carboxylic acid, the chloro-amine exchange is not selective, replacement of the fluorine taking place in a substantial extend (Patents WO 8710587, WO 8703595, Chinoin; ES 505207, Kyorin). The diazonium 6-hexafluorophosphate of a 8-azaquinolone gives a mediocre yield of the 6-fluoro derivative. Other fluorination attempts employing Balz-Schiemann method have failed (Matsumoto et al., J.Med.Chem., 27, 292-301, 1984).

The most utilized cyclization agents display however significant technological drawbacks. Diphenylether and Dowtherm are generally employed at high temperature, and they require therefore significant energy consumption in both the relevant heating and cooling processes. In addition, in order to reach the required temperatures the ethanol liberated in the cyclization process has to be distilled off, and therefore when rapid heating to 200-250°C is effected the dangerous problems of its flammability and accummulation arise. The PPA is a viscous, protic, non-recoverable solvent. Its use is limited to cyclization of tertiary-nitrogen compounds at relatively high temperatures but it is incompatible with the N-trimethylsilyl and other functional groups such as the cyano. The PPE has, among other inconveniences, its costly preparation, and the extended reaction times it requires (1.5 to 3.0 days).

The subject matter of the invention here claimed is novel organosilylpolyphosphoric reagents, their preparation procedure and their use in technological processes for the synthesis of quinolone-3-carboxylic acids. The problems arising from the classical methods of synthesis involving the sequence:

(A) AMINE ---→ (B) AMME ---→ (C) QUINOLONE,

are in sharp contrast with the advantages of the invention here described which uses these new reagents in the different stages of the process. Precisely (A) an amine or N-trimethylsilylamine, (B) a N-trimethylsilyloxymethylenemalonate or an aminomethylenemalonate, and (C) an organosilylpolyphosphate for the cyclization. For the first time, organosilyl compounds are used in the preparation of quinolones with most remarkable results.

It has been discovered that the cyclodealkylation reaction is performed in excellent results by means of a highgly reactive agent the molecular composition of which is given by formula I,

$[P_2O_5]_N \cdot [Y[(R^0)_3Si]R^1]$ (I)

where $1 < N < 10$; Y being an oxygen atom, two chlorine o two bromine atoms; $R^0$, a C1-C4 alkyl group, either equal or different; and $R^1$ equal to $R^0$ or a $(R^0)_3Si$.

Compounds of formula I, which are here called either silyl (SPPS) or alkylsilyl (SPPAS) superphosphates include a new class of products characterized by their high contain in monomeric phosphorus pentoxide ($P_2O_5$) units. Given an amount of phosphorus pentoxide, the value N determines the proportion of alkylsiloxane involved in the reagent resulting from the reaction of phosphorus pentoxide with a siloxane or an alkyloxysilane.

The reaction between phosphorus petoxide and a siloxane is extensively described in the scientific and technical literature, particularly with hexaalkyldisiloxane as reported by Voronkov (Zh.Obsch.Khim., 25, 473, 1955); Chem.Abstr., 50, 2418, 1956), Schmidt et al. (Chem.Ber., 93, 872, 1960) and Imamoto (J.Synthe.Org. Chem.Japan, 43(12), 1164-1167, 1985). The latter author describe the preparation and applications of trimethylsilyl polyphosphate (PPSE) reported previously (Tetrahedron Lett., 22, 1803, 1981).

Polyorganylphophosilanes have also been commented by M.G. Voronkov, V.P. Mileshkevich and Yu.A. Yuzhelevskii ("The Siloxane Bond", Trad. J. Livak, Consultants Bureau, New York, 1978). Triorganylsilylphosphates result from the reaction of trialkylsilanoles with orthophosphoric acid (page 381) or with phoschorus pentoxide (page 382), and from alkoxysilanes and phosphorus petoxide (page 244). Polyorganylphosphosiloxanes result from treatment of the corresponding polyorganosiloxane-alpha, omega-diols or polyalkyloxysilanes with phosphorus pentoxide. Also, reference is made of the tris-trimethylsilylphosphate by R.S. Edmundson ("Dictionary of Organophosphorus Compounds", Chapman and Hall, London, 1988, page 872) and D.E.C. Corbridge ("Phosphorus", third eddition, Elsevier, Amsterdam, 1985, page 500).

A review on PPSE including its applications has been published by T. Imamoto (Journal of Synthetic Organic Chemistry, Japan, vol. 43, no. 12, 1985; in japanese).

As far as the present invention, the trialkylphosphates, polyorganylphosphosilanes and the trimethylsilyl plyphosphate (PPSE) itself are adequate, although phosphorus pentoxide is regarded as preferential since it allows easy control and reproduction for the fast formation of new reagents of formula I.

For the preparation of the reagent according to the invnetion here claimed, given the amount of phosphorus pentoxide ($P_2O_5$, monomer) the proportion of siloxane becomes the variable of the process. Table 1, Example 2 reports the results reached in the simple, practical case of the hexamethyldisiloxane (HMDS).

In such a case, variable HMDS is shown as a function of N, being $1 < N < 10$. N besides its being a fundamental parameter, it characterizes, differenciates and controls the SPPS activity in the SPPS/P system (P = compound to be cyclized) versus temperature and reaction time.

The composition of PPSE was established by Watanabe (Chem.Lett., 1225, 1982). It consists predominantly in a mixture of tetratrimethylsilyl isocyclotetraphosphate and cyclotetraphosphate, and hexatrimethylsilyl tetraphosphate, along with a minor proportion of tetrakistrimethylsilyl pyrophosphate. The relative proportion of these compounds are N = 1 to 0.66, and referred to the reagent, N = 0.56 for the PPSE and N = 0.16 for the tris(trimethylsilyl)phosphate.

Example 5, Tables 1, 2 and 3 display the results for N = 0.56 (PPSE), N = 0 (HMDS) and N = $\infty$ - ($P_2O_5$). Same Example, Table 5, gives the relevant yields for the cyclization with phosphorus pentoxide which, along with those reported in the literature, reveal a lack of efficiency. Same Example, Table 4 displays the remarkable results with reagent SPPS.

The structure of the new organosilylpolyphosphates, such as the trimethylsilylsuperpolyphosphate (SPPS), is constituted by primary monomeric phosphorus pentoxide units and secondary polymeric subunits which contribute to the cyclopolyphosphate structure. It is pointed out that the values of N would be specific for a class of trimethylsilyl cyclosuperpolyphosphates which in turn may originate polymers which would undergo with temperature expansions or compressions modifying their reactivity (Example 5, Tables 1, 2, 3 and 4). By definition, 1 mol/g of SPPS is equivalent to 1 mol/g of $P_2O_5$ monomer.

The preparation of a SPPS or SPPAS reagent system represented by formula I may be effected in an organic solvent according to either of methods: (a) with $P_2O_5$ and a siloxane, (b) $P_20_5$ with a trialkylsilyl polyphosphate (PPSE) or an alkyltrialkylsilyl polyphosphate (PPASE).

These and other remarkable results constitute the foundation of product and process new technologies in the field of the 1-substituted 1,4-dihydro-4-oxo-3-pyridinecarboxylic and 4-hydroxy-3-pyridinecarboxylic acids, and derivatives.

According the invention here claimed, the preparation of a complex represented by formula I is effected by the reaction of phosphorus pentoxide with, preferently, chlorotrimethylsilane, bromotrimethylsilane, an hexaalkyldisiloxane, such as hexamethyldisiloxane or an alkoxytrialkylsilane, such as methoxytrimethylsilane, ethoxytrimethylsilane, a butoxytrimethylsilane or terbutoxytrimethylsilane, at temperatures ranging from 20° to 135°C, in an organic solvent. At hot temperatures, a solution is obtained which by concentration and cooling separates the reagent as a very hygroscopic solid. Therefore, for practical reasons the reagent is not isolated but used directly (method a). When using PPSE, obviously the direct use of the quantities defined by the selected value of N is preferred. For this reason, method (b) would be a particular situation.

Preferred inert organic solvents include dichloromethane, chloroform, carbon tetrachloride, dichloroethane, trichloroethylene, benzene, toluene, xylenes, chlorobenzenes, dichlorobenzenes -particularly o-dichlorobenzene-, nitrobenzene, etc.. Also, current low-price industrial ethers such as 1,4-dioxane and diphenylether. All them allow the preparation of formula I compound solutions. Preferably, common, easily recoverable solvents are used which, in terms of the temperature to be reached, are fit for the preparation of the relevant SPPS.

The resulting solutions are useful in the preparation of quinolones, and azaquinolones and their pharmacologically acceptable salts. It has now been discovered that by means of a solution containing a formula I reagent excellent results and a generalized application to the cyclodealkylation of enamines (N-substituted dialkyl aminomethylenemalonates) of formula II are reached

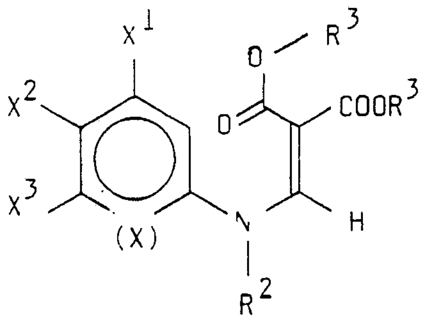

(II)

where (X) can be a trivalent species such as N, C-H, C-$NO_2$, C-OH, C-COOH, C-$COOCH_3$; $X^1$ , $X^2$ and $X^3$ are selected among hydrogen, fluorine, chlorine, bromine, C1-C4 alkyl, nitro, cyano, sulfonic, carboxyl, C1-C5 carboxyalkyl, hydroxyl, methoxyl, methylenedioxy, C1-C4 aminodialkyl, 1-piperazinyl, 4-alkyl(C1-C4)-1-piperazinyl, 4-acyl-1-piperazinyl with C1-C8 acyl, di(beta-hydroxyethyl)amino, di(beta-chloroethyl)amino, aryl, substituted aryl, benzyl, substituted benzyl or doubled benzyls forming an alicyclic ring, aromatic, heterocyclic or a system of condensed rings; $R^2$ a monovalent species among hydrogen, hydroxyl, trimethylsilyl, C1-C4 alkyl, C3-C7 cycloalkyl, cyclopropylmethyl, aryl, benzyl, C1-C4 aminodialkyl, or bonded to (X), the latter being a carbon atom belonging to a cyclic or heterocyclic system, preferably a six link system as the oxazine, thiazine, piperazine, piperidine or hydropyridine rings; $R^3$ a straight chain C1-C5 alkyl, isopropyl, terbutyl, benzyl, trimethylsilyl, or a hydrogen atom for the formation of a quinolone or azaquinolone of formula III

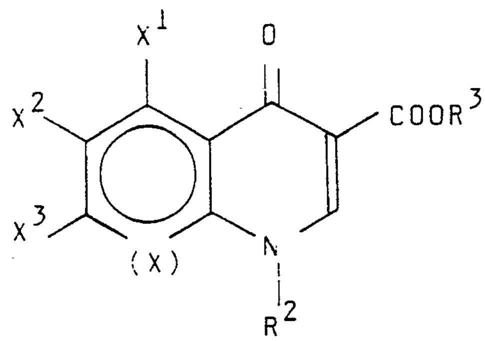

(III)

where (X), $X^1$, $X^2$, $X^3$, $R^2$ and $R^3$ mean as above.

As far as the invention here described is concerned, the cyclization reaction may be carried out according to either (a) or (b). The molar ratio between the reagent (given as monomeric $P_2O_5$) and the product formula II (P) may be altered according to the secondary o tertiary amine, and the nature and influence of the substituents $X^1$, $X^2$, $X^3$ and $R^2$. This ratio is (SPPS):P, which may vary from 0.5 to 8.0, preferably between 2.0 and 5.0. The reaction times which depend upon the temperature, vary from 5 min. to 24 h., preferably between 5 and 60 min.

Compounds of formula II are prepared by conventional methods described in the cientific and technical literature. The difficulties inherent to the method, due to the nature of the $R^2$-$NH_2$ group and the purity of the ethoxymethylenemalonate, influence the isolation process, the yield and the purity (preceding references). For the purpose of the invention here described, in connection of the improvement of the relevant technological aspects through the use of reagents SPPS or SPPAS, and the selection of the most appropriate preparation method, the following new procedural options have been adopted consisting in the selection of product of formula IV

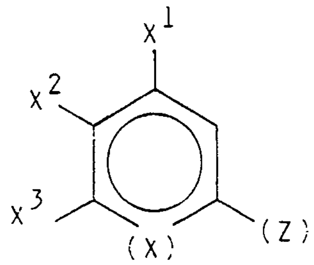

(IV)

where (X), $X^1$, $X^2$ and $X^3$ have the meaning given before. IV is made to react with a methylenemalonate of formula V,

$(Z^1)$-CH = $(COOR^3)_2$ (V)

where $R^3$ has the aforesaid meaning, and when $(Z^1)$ is a $N(R^2)H$ group, being $R^2$ as before, (Z) in formula IV is selected among a chlorine or bromine atom. When $(Z^1)$ is a hydroxyl, methoxyl, ethoxyl, trimethylsilyloxy, chloro, p-toluenesulphonate or methanesulphonate, in formula IV (Z) represents a $N(R^2)H$ group, with the meaning assigned to $R^2$.

Scheme 1 represents a preparation sequence for a compound of formula II involving the cyclization reaction. The dialkyl trimethylsilyloxymethylenemalonate (VI), such as the diethyl ester (SMME), ensues easily from the reaction between formylmalonate or hydroxymethylenemalonate (HMME) and a silylating agent.

The reaction of formylmalonic acid, $(Z^1)$ = OH and $R^3$ = H in formula V, with a silylating agent results in the formation of trimethylsilyl trimethylsilyloxymethylenemalonate (VII).

Scheme 1

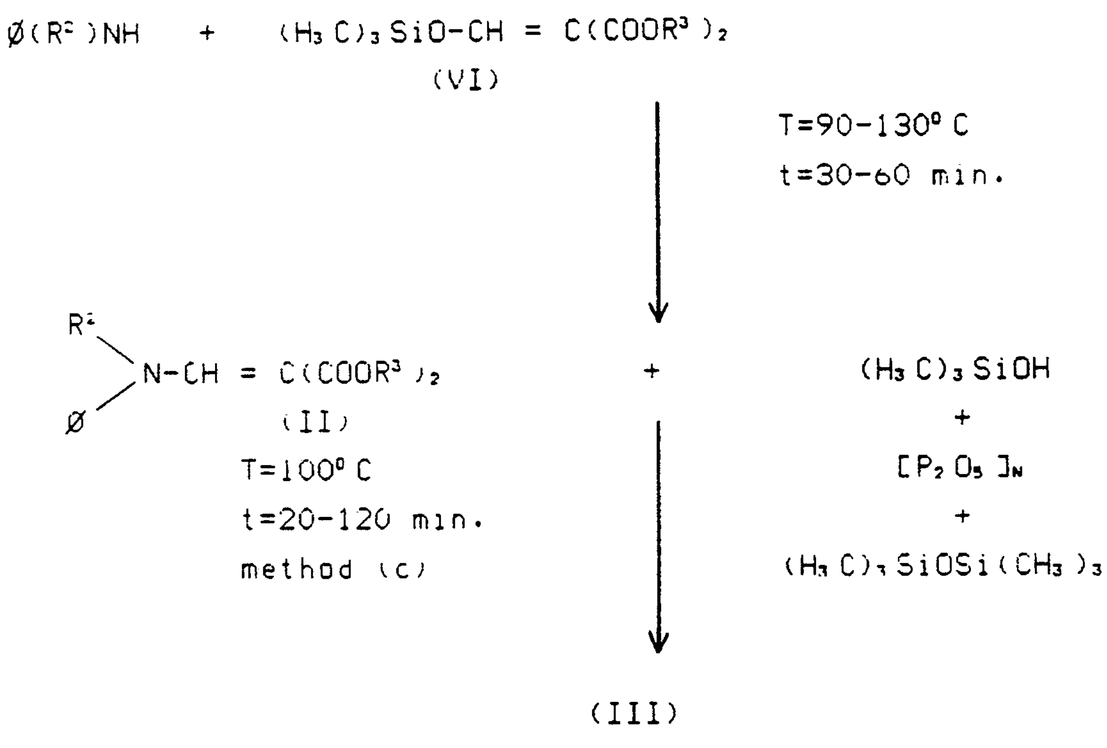


(VI)

(II)

(III)

CYCLIZATION

A useful alternative consists in the alcoholysis of a aminomethylenmalonitrile prepared according to Ceder and Vermark method (Acta Chemica Scandinavica B, 31, 235-238, 1977) to give compounds of formula II.

The silylating agents include preferentially the group constituted by trimethylchlorosilane, hexamethyl-

disilazane (HMDS), 3-trimethylsilyl-2-oxazolidinone (TMSO), trimethylsilyl triflate, dimethylisopropylchlorosilane, tripropylchlorosilane, dimethylterbutylchlorosilane, triphenylchlorosilane and other well-known (A.E. Pierce, "Silylation of Organic Compounds", Pierce Chem. Comp., Rockford, Illinois, 1968).

The diethyl formylmalonate is obtained as described in the literature (Claisen, Annalen, 297, 78, 1897); the formylmalonic acid or its alkaline or alkaline-earth salts, by Ruhemann and Morrell method (Soc., 59, 749) or by formylation in ethanol with diethyl malonate in the presence of one equivalent of sodium methoxide.

When $R^3$ is a hydrogen atom, compounds of formula III are quinolone-, azaquinolone- or diazaquinolonecarboxylic acids, the aromatic ring of the latter being substituted by a pyridazine ring. Also, the cyclic derivatives where the (X)-N-$R^2$ system forms a ring. Their preparation is conveniently effected by basic or acid hydrolysis, or by acidolysis with hydrogen-chloride/1,4-dioxane. In the first case, the hydrolysis is carried out by heating between 40 and 100 °C, and adjusting afterwards at PH = 6-7; in the second case, the quinolone hydrochloride is formed. Generally, the acid compound results as a precipitate directly by dilution with water of the reaction mass. In any case, the isolation of the formula III product ($R^3$ = H) is achieved by conventional filtration methods. When the cyclization reaction is carried out with a mixture of the ester and acid, being $R^3$ = alkyl and $R^3$ = H, their separation is performed by solubilization of the acid in alkaline media, being then recovered by precipitation at pH = 6-7.

Salts of compounds of formula III, being $R^3$ = H, may be conveniently prepared from the corresponding acid: sulfonic, hydrochloric, sulfuric, phosphoric, or aliphatic, aromatic, alkanearomatic or heterocyclic carboxylic acids. For this purpose, a solution of the selected acid in methanol is added to another solution of compound of formula III, $R^3$ = trimethylsilyl, in an inert solvent. Adequate inert solvent are 1,4-dioxane, chloroform and dichloromethene, and other. The preferred acids are those forming pharmaceutically admissible salts.

## Example 1

### Trimethylsilyl superpolyphosphate (SPPS)

A. To the selected solvent (D; 2, 5, 10 or 15 ml), first phosphorus pentoxide (5.68 g; 4.0 cmole, calculated as monomer), and next hexamethyldisoloxane (4.16 ml; 2.0 cmole) are added. The mixture is warmed while stirring, and about 50-55 °C an exothermicity appears the extent of which depends upon the reagents concentration. The desired temperature (45 to 130 °C, depending upon concentration and solvent) is kept for a while (15 to 30 min). The reaction mass is slightly turbid solution. In chlorobenzene, at 130 °C, it is a milky solution which by standing releases a sediment. In diphenylether or nitrobenzene at 110 °C it is a practically transparent solution, and in benzene, at 80-82 °C it becomes a gel-like slight dispersion. In dichloromethane, the temperature raises abruptly about 15 °C, and reflux temperature is reached.

The SPPS prepared this way is colled down to about 40-75 °C (avoiding moisture) and it is used next for the cycloacylation reaction of the N-substituted diethyl aminomethylenemalonate (AMME = P; 1.0 cmole).

Molar ratios: SPPS equivalent to $P_2O_5$ monomer (m.w. = 141.94), $P_2O_5$/HMDS = N, and SPPS/P. The ratio D/SPPS, in ml of solvent (D) per gram of superpolyphosphate (SPPS).

B. Involving preparation in situ of the trimethylsilyl polyphosphate (PPSE): To a suspension of phosphorus petoxide (1.58 g; 1.12 cmole $P_2O_5$) in the solvent of choice (D, as in A) hexamethyldisiloxane (4.16 ml; 2.0 cmole) is added, and the mixture is refluxed until a solution where N = 0.56, corresponding to PPSE, is obtained. The required amount of phosphorus pentoxide (4.10 g; 2.88 cmole) is then added, proceding next as in A. A solution where N = 2.00 is obtained, which corresponds to a SPPS. The value of N may be varied by changing the amount of $P_2O_5$.

C. A suspension of $P_2O_5$ (7.10 g; 5.0 cmole) in chloroform (10 ml) containing hexamethyldisiloxane (3.57 ml; 1.67 cmole) is refluxed until total disolution (15-30 min.). The reagent turns out with N = 3, D/SPPS = 2 ml/cmol being SPPS/P value coincident with that established for P (product to cyclize).

## Example 2

### Trialkylsilyl superpolyphosphate

Following Example 1, sections A, B or C, the hexamethyldisiloxane is substituted by an equivalent amount of hexaalkyldisiloxane, extending the refluxing time, or, when needed, using a higher boiling solvent. Hexaethyldisiloxane, hexapropylsiloxane, tetramethylditerbutyldisiloxane, tetramethyldiisopropyldisiloxane and n-hexabutyldisiloxane are preferable.

Example 3

Trimethylchlorosilyl superpolyphosphates (Cl-SPPS)

To the adopted solvent (D; 2, 5, 10 or 15 ml, chlorobenzene preferentially) is added first phosphorus pentoxide (5.68 g; 4.0 cmol), and then trimethylchlorosilane (2.50 ml; 2.0 cmole); N = 2. The mixture is refluxed until a constant reflux temperature is reached (90 min., chlorobenzene). The slightly turbid resulting solution is preserved from the air moisture. When trimethylbromosilane is used, Br-SPPS is obtained. Equivalent results are obtained when using dimethyldichlorosilane.

Example 4

Alkyltrimethylsilyl superpolyphosphates (SPPAS)

A. Isopropyltrimethylsilyl superpolyphosphate (IP-SPPS). To the adopted solvent (D; 2, 5, 10 or 15 ml) is added fisrt phosphorus pentoxide (5.68 g; 4.0 cmole) and then isopropyloxytrimethylsilane (2.64 g.; 2.0 cmole). The resulting mixture is for a time (15 to 30 min.) heated up to either the desired temperature or to reflux. A slightly turbid solution of the reagent is obtained which is being preserved from the air moisture and characterized by the following molar ratios: IP-SPPAS equivalent to $P_2O_5$, $P_2O_5$/isopropyloxytrimethylsilane = N and IP-SPPAS/P; D/IP-SPPAS (ml/cmol).

B. Alkyltrimethylsilyl superpolyphosphates. Following the example 4A, and using chlorobenzene as the solvent (D; 5 or 10 ml), but substituting the isopropyloxytrimethylsilane by the proper aamount of methyloxytrimethylsilane, methyloxytriethylsilane, methyloxytributylsilane, terbutyloxytrimethylsilane, propyloxytrimethylsilane, or a compound selected among the following ones:

$(H_3C)_3Si\text{-}OCH_2O\text{-}Si(CH_3)_3$

$(H_3C)_3Si\text{-}OCH_2CH_2O\text{-}Si(CH_3)_3$

$(H_3C)_3Si\text{-}OCH_2CH_2O\text{-}CH_3$

$(H_3C)_3SiO\text{-}CH_2CH_2[OSi(CH_3)_3]CH_2\text{-}OSi(CH_3)_3$

$(H_3C)_3Si\text{-}OCH_2CH_2[OSi(CH_3)_3]CH_3$

$(H_3C)_3Si\text{-}OCH_2CH_2O\text{-}CH_2CH_3$

$(H_3C)_3Si\text{-}OCH_2CH_2CH_2O\text{-}Si(CH_3)_3$

to settle reagent parameter N.

Example 5

N = f(temperature, time and solvent)

Tables 1, 2 and 3 display the data for different values of N concerning the composition of SPPS and SPPAS, and the pertinent results. The reagent are prepared according to example 1A in chlorobenzene, 1C for chloroform, and 4 for SPPAS varying the silane. The maximal reagent efficiency corresponds to $2 < N < 8$, showing the influen ce of the reaction time. When N -> 0 the activity approaches to zero, and in the case of the trimethylsilyl polyphosphate (PPSE) it becomes ineffectual (N = 0.56). When N-> ∞ the activity is that of the phosphorus pentoxide, with the limitations due to the nature of the amine.

In table 4 the results concerning some secondary and tertiary amines with reagent SPPS, using different solvents and temperatures, are summarized. Comparing these results with those of table 5 including the use of phosphorus pentoxide, PPA and DFE, the remarkable role of reagent SPPS, and the influence of N are made evident. The examples nos. 1 and 2 compare with those of 14 and 21; 3 with 12: 7

with 13. Nos. 4 with 16 and 17; 5 with 15 and 18; 6 with 19; 8 with 20. These results lead to an obvious conclusion.

As far as the phosphorus pentoxide is concerned. Makees et al. (loc. cit.) prescribe, when the reaction is over, the addition of a small proportion of water. The authors of this invention have corroborated the formation of polyphosphoric acid. According to Makees et al., the PPA was used to cyclize the less reactive tertiary aminomalonates; i. e., those giving very low yields.

An important fact about phosphorus pentoxide, conveying an unprecendented technological difficulty, centers in the cyclization reactions described in the literature and checked by the authors of the invention here described. It is the existence of a minimal temperature to iniciate the reaction, which proceeds next with an abrupt increase of temperature (160-190 °C).

Comparison of examples nos. 9 and 10 (Table 4) with 22 and 23 (Table 5) is quite meaningful, being particlarly relevant the performance of reagent SPPS, in dichloromethane at room temperature, versus phosphorus pentoxide, no. 11 (Table 4) and no. 24 (Table 5).

Table 1

| Cyclization: Diethyl (4-fluoro-3-chloroanilino)methylenemalonate (P) | | | | |
|---|---|---|---|---|
| HMDS (cmol) | N | t (min.) | C (%)* | A (%)* |
| 2.00 | 0.00 | 360 | 0 | 0.0 |
| 7.14 | 0.56 | 360 | < 60 | 4.0 |
| 2.67 | 1.50 | 60 | > 99 | 86.5 |
| 2.00 | 2.00 | 15 | > 99 | 90.3 |
| 1.33 | 3.00 | 15 | > 99 | 88.5 |
| 1.00 | 4.00 | 15 | > 99 | 87.8 |
| 0.50 | 8.00 | 15 | > 99 | 87.0 |
| 0.00 | ∞ | 120 | > 99 | 48.0 |

* C = % conversion of P (C.C.F.); A = % of isolated ester. Conditions: P = 1 cmole; D = chlorobenzene (10 ml); T = 130 °C; SPPS/P = 4 ($P_2O_5$ = 4 cmole). HMDS = hexamethyldisiloxane.

Table 2

| Cycloacylation: Diethyl (N-ethylanilino)methylenemalonate (P) | | | | |
|---|---|---|---|---|
| HMDS (cmol) | N | t (min.) | C (%)* | A (%)* |
| 2.00 | 0.00 | 360 | 0.0 | 0.0 |
| 8.93 | 0.56 | 360 | 30.0 | 10.0 |
| 5.00 | 1.00 | 240 | ≈60.0 | 55.0 |
| 2.50 | 2.00 | 120 | > 99 | 93.0 |
| 1.67 | 3.00 | 60 | > 99 | 96.0 |
| 1.25 | 4.00 | 60 | > 99 | 95.0 |
| 0.62 | 8.00 | 75 | > 99 | 96.0 |
| 0.00 | ∞ | 360 | 20.0 | 16.0 |

* C = % conversion of P (CCF); A = % of isolated acid. Conditions: P = 1 cmole; D = Chloroform (10 ml); T = 62-65° C; SPPS/P = 5 ($P_2O_5$ = 5 cmole). HMDS = hexamethyldisiloxane.

Table 3

| Cycloacylation: Diethyl (4-fluoro-3-chloroanilino)methylenemalonate (P) | | | |
|---|---|---|---|
| Silane (SPPAS) | t (min.) | C (%)* | A (%)* |
| IPS | 15 | > 99 | 84.5 |
| MS | 15 | > 99 | 80.7 |
| n-BS | 15 | > 99 | 86.8 |
| TMCIS | 15 | > 99 | 85.4 |

Conditions: P = 1.0 cmole; silane = 2.0 cmole; D = chlorobenzene (10 ml); T = 130° C; $P_2O_5$ = 5.0 cmole; N = 2; Cl-SPPS and SPPAS are synthesized as in examples 3 and 4, respectively. IPS = isopropyloxytrimethylsilane MS = methyloxytrimethylsilane; n-BS = n-butyloxytrimethylsilane; TMCIS = trimethylchlorosilane.

Table 4. Cycloacylation: $RR^2NCH = C(CO_2C_2H_5)_2$ (P)

| N° | R | $R^2$ | D | N | $\frac{SPPS}{P}$ | $\frac{D(ml)}{SPPS}$ | T(°C) | t(min) | R(%) |
|---|---|---|---|---|---|---|---|---|---|
| 1 | 4F,3ClØ | H | CLB | 2 | 4 | 2.50 | 130 | 15 | 90.0 |
| 2 | 4F,3ClØ | H | NBC | 2 | 4 | 2.50 | 130 | 15 | 87.0 |
| 3 | 4F,3ClØ | H | BEC | 2 | 8 | 1.25 | 82 | 120 | 88.5 |
| 4 | Ø | $C_2H_5$ | CLB | 3 | 4 | 2.50 | 112 | 10 | 96.0 |
| 5 | Ø | $C_2H_5$ | CLF | 3 | 5 | 2.00 | 70 | 35 | 95.0 |
| 6 | Ø | $(CH_3)_2CH$ | CLF | 3 | 5 | 2.00 | 70 | 35 | 96.0 |
| 7 | 4F,3ClØ | H | BEC | 2 | 8 | 1.25 | 95 | 60 | 86.5 |
| 8 | o-ClØ | $C_2H_5$ | CLF | 3 | 5 | 2.00 | 70 | 60 | 93.0 |
| 9 | 6-$CH_3$Py. | H | CLB | 3 | 4 | 2.50 | 130 | 20 | 90.0 |
| 10 | α-Naf. | H | CLB | 2 | 4 | 2.50 | 130 | 15 | 88.0 |
| 11 | Ø | $C_2H_5$ | DCM | 3 | 6 | 1.67 | 25 | 22(h) | 87.0 |

CLB (chlorobenzene), NBC (nitrobenzene), BEC (benzene), CLF (chloroform), DCM (dichloromethane), Py = 2-pyridyl, Ø = aryl, Naph = Naphthyl. 4, 5, 6 and 8 are isolated as carboxylic acids.

Table 5. Cycloacylation: $RR^2NCH = C(CO_2C_2H_5)$ (P)

| N° | R | $R^2$ | D | RT | $\frac{RT}{P}$ | $\frac{D(ml)}{RT}$ | T(°C) | t(min) | R(%) |
|---|---|---|---|---|---|---|---|---|---|
| 12 | 4F,3ClØ | H | BEC | $P_2O_5$ | 8 | 2.25 | 80 | 125 | 4.0 |
| 13 | 4F,3ClØ | H | BCL | $P_2O_5$ | 8 | 1.25 | 95 | 60 | 9.0 |
| 14 | 4F,3ClØ | H | DFE | DFE | - | -- | 250 | 60 | 70.0 |
| 15 | Ø | $C_2H_5$ | BEC | $P_2O_5$ | 2 | 30 | 80 | 240 | 17.0 |
| 16 | Ø | $C_2H_5$ | DFE | --- | - | -- | --- | --- | 0.0 |
| 17 | Ø | $C_2H_5$ | PPA | PPA | 10 | 1.0 | 120 | 15 | 60.0 |
| 18 | Ø | $C_2H_5$ | CLF | $P_2O_5$ | 5 | 2.0 | 63 | 60 | 6.0 |
| 19 | Ø | $(CH_3)_2CH$ | CLF | $P_2O_5$ | 5 | 2.0 | 63 | 60 | 5.0 |
| 20 | o-ClØ | $C_2H_5$ | NBC | $P_2O_5$ | 2.3 | 2.0 | 160-190 | 15 | 16.0 |
| 21 | 4F,3ClØ | H | NBC | $P_2O_5$ | 2.2 | 2.0 | 160-190 | 10 | 55.0 |
| 22 | 6-$CH_3$Py | H | NBC | $P_2O_5$ | 2.2 | 2.0 | 160-190 | 10 | 25.5 |
| 23 | α-Naf | H | CLB | $P_2O_5$ | 2.2 | 2.0 | 130 | 120 | 45.0 |
| 24 | Ø | $C_2H_5$ | DCM | $P_2O_5$ | 6 | 1.67 | 25 | 22(h) | 17.0 |

RT (reagent), BCL (benzene-chlorobenzene), DFE (diphenylether), PPA (polyphosphoric acid). (14) Koga et al., J.Med.Chem. 23, 1358-1363, 1980. (15, 16 and 17) Nakagome et al., J.Heter.Chem., 8, 357, 1971, (20) Markees et al., J.Med.Chem., 17, 137-139, 1974. 14, 15 and 17 are isolated as esters; the rest, as carboxylic acids.

Example 6

Preparation of intermediates

A. Diethyl trimethylsilyloxymethylenemalonate (SMME)

To a solution of diethyl hydroxymethylenemalunate (18.18 g; 0.1 mole) in carbon tetrachloride (100 ml) at room temperature 3-trimethylsilyl-2-oxazolidone (TMSO; 16.0 g; 0.1 mole) and triethylamine (0.5 ml; 0.36 cmole) are added. Immediately, a moderately hexothermic reaction take place, and after a while 2-oxazolidone precipitates. The resulting mixture is stirred for 60 min., and after filtration in an inert atmosphere and evaporation under vacuum gives the title compound (SMME; 26.0 g) as an oil in a practically quantitative yield. The spectrum $H^1$-NMR shows the peaks due to both the $CH_3$-Si and $CH_3$-O arrangements. The product does not decompose by distillation under vacuum, and it is perfectly stable under ordinary conditions. The dimethyl trimethylsilyloxymethylenemalonate (SMMM) is obtained starting from the corresponding dimethyl malonate.

The same results are achieved replacing the triethylamine by DBU.

B. Dimethyl trimethylsilyloxymethylenemalonate (SMMM)

To a stirred, cooled (0-5°C) suspension of TMSO (16.0 g; 0.1 mole) in dimethyl hydroxymethylenemalonate (16.0 g; 0.1 mole) trifluoromethanesulfonic acid (0.5 ml) is added. The stirring is

continued until room temperature is reached (20-25° C), and the carbon tetrachloride (100 ml) is added and the 2-oxazolidinone is filtered off. Vacuum elimination of the solvent from the filtered solution affords as an oil the title compound (SMMM) in a virtually quantitative yield (23.0 g).

C. Ditrimethylsilyl trimethylsilyloxymethylenemalonate (SMMS)

A solution of TMSO (48.0 g; 0.3 mole), formylmalonic acid (13.21 g; 0.1 mole) and trifluoromethanesulfonic acid (0.5 ml) in carbon tetrachloride (100 ml) is refluxed gently and stirred during a short time (30 min.). Working up the reaction mass as in the preceding cases, the title compound (SMMS) is obtained as an oil in a virtually quantitative yield (34.3 g). It is kept from moisture.

D. Diethyl N-cyclopropylaminomethylenemalonate

To cyclopropylamine (5.71 g; 0.1 mole), cooled down to 0-5° C, diethyl ethoxymethylenemalonate (21.62 g; 0.1 mole) is gradually added (30 min.) while stirring, letting finally the system reach 20° C. Next, the ethanol formed is distilled off (about 60° C) under vacuum. The title compound (22.3 g) is obtained as an oil (colorless, after vacuum distillation) in a 98.1% yield; d = 1.109 (20° C). $n_D$ = 1.5133 (24° C). Distilled in Kugelrohr (Buchi GKR-50): b.p. = 150° C (0.15 torr), I.R. (KBr-film) $\nu$, 3260 (NH). 2970 ($C_2H_5$), 1710, 1680, 1650, 1600 $cm^{-1}$.

E. N-substituted anilines

To the Schiff-base (0.1 mole), prepared optionally "in situ" (0.1 mole each component), suspended in ethanol (150 ml), acetic acid (5.7 ml; 0.1 mole) is added. Next, at 0-5° C, with stirring, sodium borohydride (3.8 g; 0.1 cmole) is stepwise added during 30 min.. After stirring for an additional 30 min. at 0-5° C, water is added (300 ml), the temperature raising to 20-25° C. Next, the pH of the mass is adjusted to 7 (with a 20% w/v NaOH aqueous solution). In some cases the resulting amine is separated by filtration, dissolved in dichloromethane, and the resulting solution is dried with sodium sulfate and its solvent evaporated under vacuum. In other cases, the (liquid) amine is extracted with dichloromethane and proceeding in the same manner the corresponding N-alkylaniline is obtained, the yield varying from 87 to 95% of theory (among other, N-benzylaniline, N-benzyl-α-naphthylamine, N-cyclopropyl-4-fluoro-3-chloroaniline, N-benzyl-4-fluoro-3-chloroaniline, N-benzyl-6-methyl-2-aminopyridine). The corresponding azomethines are obtained by the reaction between the aldehyde or the ketone, and the aromatic amine, as described in the literature.

In the case of N-isopropylanilines, the reaction was carried out as described before. For the Schiff base from acetone, prepared "in situ", an excess of the latter was used.

F. N-cyclopropyl-4-fluoro-3(4-acetyl-1-piperazinyl)aniline

4-Fluoro-3-chloroaniline (1.45 g; 1.0 cmole) and N-acetylpiperazine (2.56 g; 2.0 cmole) are dissolved in dimethylformamide (20 ml), and refluxed. After total conversion, as monitored by C.C.F., the solvent is evaporated under vacuum, the residue treated with water and filtered. The solid obtained after several recrystallizations in benzene yielded 4-fluoro-3(4-acetyl-1-piperazinyl)aniline, m.p. = 130-132° C.

The product (2.37 g; 1.0 cmole) was dissolved in dimethylformamide or acetonitrile (10 ml) and a solution of trimethylsilyl α-bromocyclopropylcarboxylate in acetonitrile was added next. After stirring overnight at room temperature, in the exclusion of moisture, methanol (2 ml) was added and the mixture refluxed in the presence of DBU (1,8-diazabbicyclo[5.4.0]undec-7-ene). Next, the solvent was distilled off under vacuum, and the residue was treated with water and recrystallized from benzene. Following method I, the reaction with diethyl trimethylsilyloxymethylenemalonate yields the corresponding diethyl aminomethylenemalonate.

G. N-substituted 4-Fluoro-3(1-piperazinenyl)anilines or pyrrolidine anilines

1. To a suspension of 4-fluoro-3(4-acetyl-1-piperazinyl)-aniline (4.74 g: 2.0 cmole) in water (50 ml), in

the presence of sodium hydroxide (0.6 g; 1.5 cmole) p-toluenesulfonyl chloride (2.28 g; 1.2 cmole) is stepwise added, with temperature control (40-45 °C). After a while (30-60 min.), the pH is adjusted to 6.0, the sulfamide formed separated by filtration and dried (quantitative yield). The resulting solution of the sodium sulfamide (4.13 g; 1.0 mole) in dimethylformamide (10 ml) is heated to 80-140 °C after addition of one equivalent of one of the following alkylating components: ethyl bromide, methyl iodide, ethyl iodide, benzyl chloride, dimethyl sulfate, ethyl p-toluenesulfonate, cyclopropyl p-toluenesulfonate, cyclopropyl bromide, 2,4-difluorochlorobenzene, terbutyl bromide, cyclohexyl bromide, or isopropyl bromide. By acidic hydrolysis (100-120 °C) with 98% sulfuric acid containing a 30% of acetic acid the sulfate of the corresponding secondary amines are obtained, from which, by alkaline treatment, the amine are liberated. The latter are purified by treatment with p-toluenesulfonic acid in 1,4-dioxane which cause the precipitation of the corresponding salts. Treatment of these salts with sodium hydroxide affords the corresponding piperazines of title G.

2. A solution of N-p-toluenesulfonyl-4-fluoro-3-chloroaniline sodium salt (6.43 g; 2.0 cmole) in dimethylformamide is heated to 80-140 °C with two (2) equivalents of an alkylating compound of those listed in G1. Next, the resulting mixture is made to react (6-8 hours) with piperazine, N-acetylpiperazine, N-methylpiperazine, N-ethylpiperazine, N-isopropylpiperazine, pyrrolidine or N-acetylaminopyrrolidine. Finally, the acidic hydrolysis and the isolation of the title compounds are carried out as in G1.

3. To a solution of N-p-toluenesulfonylcyclopropylamine sodium (or potassium) salt (4.66 g; 2.0 cmole) in dimethylformamide (20 ml) 2,4-dichlorofluorobenzene (4.12 g; 2.5 cmole) are added, and the mass is heated to 80-140 °C. The end of the reaction is ascertained by thin-layer chromatography, and then three (3) equivalents of piperazine are added and the mixture is refluxed during 6 hours. The isolated reaction product is hydrolyzed with 98% sulfuric acid containing a 30% of acetic acid and the pH is adjusted to 6. The resulting precipitate is extracted with chloroform, dried and by distilling off the solvent 4-fluor-(1-piperazinyl)-N-cyclopropylaniline is obtained. The same results are reached with N-benzenesulphonylcyclopropylamine.

4. The above-mentioned N-p-toluenesulfonyl derivatives are prepared by the reaction of p-toluenesulfonyl chloride (1.2 equivalents) and the corresponding amine (1.0 equivalents) in methanol/water. To the mixture, either at room temperature or somewhat higher (40 °C), an aqueous solution of sodium hydroxide (1.3 equivalents) is gradually added while stirring until constant alkaline pH, the latter is adjusted is adjusted to 5-6 with acetic or hydrochloric acid, and so the rest of sulfonylamine precipitates. When required, by evaporation of the methanol, filtration, washing and drying the corresponding cyclopropylamine, 4-fluoro-3-chloroaniline or 4-fluoro-3-(4-acetyl-1-piperazinyl)aniline derivatives are obtained. The same results are reached with benzenesulfonyl chloride. The dimethylformamide solution of these products treated with an equivalent of sodium methoxide give the sodium salts. Analogously, the sodium salts precipitate from methanol and from isopropanol, being isolated by filtration.

H. N-substituted diethyl 4-fluoro-3-(1-piperazinyl)anilinomethylenemalonate

A solution of diethyl (4-fluoro-3-chloroanilino)methylenemalonate (3.15 g; 1.0 cmole) in dimethylformamide (10-15 ml) with DBU (1.8 g; 1.2 cmole) and cyclopropyl bromide is stirred first at room temperature and then at 80 °C. The solvent is eliminated under vacuum, and water is added to the resulting concentrated solution. After extraction with chloroform, drying and evaporation, a residue is obtained, which is dissolved in dimethylformamide (15 ml). To the resulting solution piperazine is added (2.58 g; 3.0 cmole) and then refluxed for a while (5 hours). Finally, the residue obtained by evaporation is treated with water, filtered, and recrystallized from dichloromethane/n-hexane (or cyclohexane). Using the akylating agents mentioned in G1, the corresponding compounds of title H are obtained.

I. N-substituted diethyl aminomethylenmalonates

a) A solution of diethyl trimethylsilyloxymethylene malonate (1.0 cmole) in dichloromethane, chloroform or acetonitrile (5 to 10 ml) and the amine (1.0 cmole), with either one equivalent of magnesium sulfate of 3-trimethylsilyloxazolidinone (TMSO; 1.0 cmole) and a catalytic amount of DBU, is stirred a room temperature and later refluxed. The solvent is distilled off under vacuum and the residue is dissolved in n-hexane or cyclohexane. In the case of TMSO, the residue in washed with water to eliminate the oxazolidinone.

b) To a solution of diethyl hydroxymethylenemalonate (formylmalonate; 9.41 g; 5.0 cmole) in dichloromethane or acetonitrile (25 ml), containing anhydrous magnesium sulfate (6.0 g; 5.0 cmole), the amine

(5.0 cmole) is added at once, the reaction being moderately hexothermic. Next, the reaction mass is stirred at room temperature (20-25 °C), and if the amine is not active enough, at reflux. The reaction is monitored by TLC (eluent: n-hexane/triethylamine 1:1; chromatosheet Alugram SIL G/UV 254; Macherey-Nagel).

c) The reaction of p-toluene sulfonate, methanesulfonate or diethyl chloromethylenemalonate with one equivalent amount of triethylamine, or DBU, is carried out in acetonitrile, at room temperature.

d) A mixture of 2,4-dichlorofluorobenzene (3.30 g; 2.0 cmole), diethyl N-cyclopropylaminomethylenemalonate (3.63 g; 1.6 cmole) and potassium carbonate (2.76 g; 2.0 cmole) in dimethylsulfoxide or dimethylformamide (20 ml) is heated at 140-150 °C for a time (8 h.). The solvent is next distilled off under reduced pressure, and the resulting residue is treated with water and extracted with chloroform. The organic layer, after drying and solvent elimination under vacuum, yields N-cyclopropyl (4-fluoro-3-chloro)aniline. Analogous results are obtained using 2,4-difluoroanilino-, benzylamino-, ethylamino-, isopropylamino- or terbutylamino- methylenemalonates derivatives.

Example 7

General procedure for the preparation of quinolones

A. Reagent is prepared in chlorobenzene or chloroform (5.0 or 10.0 ml per 1 cmole of P = aminomethylenemalonate) as in example 1A, with N values from 3 to 5, and ratios SPPS/P from 3 to 6. The compound to cyclize (P; 1.0 cmole) and the temperature (70-120 °C) are monitored by TLC. The times usually vary from 15 to 120 min.. In the reagent prepared from alkyloxytrimethylsilanes the ratios SPPAS/P varies between 5 and 6, and the N-value, between 2 and 5.

Compounds P derived from secondary amines o provided with functional substituents amino, hydroxyl or amide may previously be silylated in the usual ways. The results are shown in Table 6.

The isolation of the quinolinecarboxylic esters is effected by dilution of the reaction mixture either with water or with methanol (10-20 ml per 1 cmole of P). The acids are obtained by hydrolysis, as described in the relevant examples.

In the case of the esters, the crystallization is effected using chloroform/n-heptane mixtures, n-hexane, cyclohexane, or ether; in other cases, using dimethylformamide. The acids, in form of soluble in methanol DBU salts, are precipitated with acetic acid.

Table 6

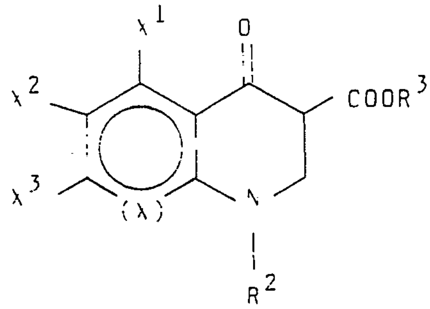

(X¹=H)

| $X^2$ | $X^3$ | (X) | $R^2$ | $R^3$ | P.F.(°C) | C* | A* |
|---|---|---|---|---|---|---|---|
| F | $HN(C_2H_4)_2N$ | N | $C_2H_5$ | H | 220-222 | 98 | 90 |
| F | F | CF | $FC_2H_4$ | $C_2H_5$ | 187-190 | 100 | 87 |
| O - $CH_2$ - O | | CH | $OC_2H_5$ | H | 258-260 | 96 | 92 |
| O - $CH_2$ - O | | CH | $OC_2H_5$ | $C_2H_5$ | 130-131 | 97 | 90 |
| F | m-ClØ$N(C_2H_4)_2N$ | CH | H | $C_2H_5$ | ----- | 100 | 96 |
| F | $H_3CN(C_2H_4)_2N$ | CF | $FC_2H_4$ | $C_2H_5$ | 163-165 | 100 | 93 |
| F | $H_3CN(C_2H_4)_2N$ | CF | $FC_2H_4$ | H | 268-269 | 99 | 93 |
| F | ØCH=CH$N(C_2H_4)_2N$ | CH | $C_2H_5$ | H | 177-190 | 98 | 88 |
| F | $H_3CN(C_2H_4)_2N$ | CH | $C_2H_5$ | H | 272-274 (1/4$H_2O$) | 95 | 92 |
| F | $HN(C_2H_4)_2N$ | CH | $C_2H_5$ | H | 220-223 | 97 | 91 |
| F | $HN(C_2H_4)_2N$ | CH | 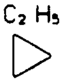 | H | 255-258 | 97 | 92 |
| F | Cl | CH | $C_2H_5$ | H | 284-285 | 95 | 90 |
| F | $HN(C_2H_4)_2N$ | CCl | 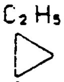 | H | >250 | 100 | 94 |
| Cl | $CH_3$ | CF | 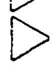 | H | 215-217 | 98 | 91 |
| F | $H_2N$ 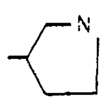 | N | 2,4-F Ø | H | >300 | 99 | 92 |
| F | $HN(C_2H_4)_2N$ | CF | 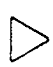 | H | 222-227 | 100 | 92 |

| | | | | | | | |
|---|---|---|---|---|---|---|---|
| F | $HN(C_2H_4)_2N$ | COH | $C_2H_5$ | H | 231-240 | 97 | 90 |
| Cl | $H_3CN(C_2H_4)N$ | CH | $C_2H_5$ | H | 257-260 | 99 | 93 |
| Cl | $O(C_2H_4)_2N$ | CH | $C_2H_5$ | H | 260-266 | 100 | 95 |
| Br | Cl | CH | H | $C_2H_5$ | >340 | 100 | 93 |
| $NO_2$ | Cl | CH | H | $C_2H_5$ | >300 | 90 | 87 |
| F | Cl | CH | H | $C_2H_5$ | 302-304 | 96 | 89 |
| F | Cl | CH | H | H | >320 | 97 | 90 |
| H | H | CH | $ØCH_2$ | $C_2H_5$ | 177-179 | 99 | 90 |
| H | H | CH | $(H_3C)_2CH$ | H | 201-204 | 99 | 96 |
| F | $HN(C_2H_4)_2N$ | N | $C_2H_5$ | H | ----- | 98 | 90 |
| H | H | CH | $ØCH_2$ | H | 234-238 | 96 | 92 |
| F | Cl | $CCH_3$ | 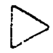 | H | 190-195 | 100 | 93 |
| O - $CH_2$ - O | | CH | H | $C_2H_5$ | 284-286 | 90 | 88 |
| O - $CH_2$ - O | | CH | $CH_3$ | H | 338-340 | 95 | 92 |
| H | H | CH | $(H_3C)_2CH$ | $C_2H_5$ | ----- | 99 | 97 |
| F | Cl | CH | $C_2H_5$ | $C_2H_5$ | 144-146 | 90 | 87 |
| F | $(C_2H_5)N(C_2H_4)_2N$ | CH | 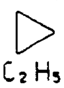 | H | 219-221 | 92 | 90 |
| H | H | CH | $C_2H_5$ | H | 254-255 | 95 | 92 |
| O - $CH_2$ - O | | CH | $(H_3C)_2CH$ | H | 294-296 | 90 | 96 |
| F | H | CF | $C_2H_5$ | H | 210-212 | 89 | 93 |
| $NO_2$ | $C_2H_5O_2CN(C_2H_4)_2N$ | CF | 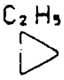 | $C_2H_5$ | ----- | 88 | 92 |
| F | $C_2H_5N(C_2H_4)_2N$ | CF | 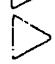 | H | 219-220 | 91 | 87 |

* C = % conversion; A = % isolation.

B. Reagent is prepared in chloroform (10.0 ml per cmole of P = aminomethylenemalonate) as in example 1A, with N = 3 and and SPPS/P = 5. The compound to cyclize (P; 1.0 cmole) is added and the mass is refluxed (70-75° C). The reaction, which is monitored by TLC, is over after 30-60 min..

The ester are isolated by pouring the resulting reaction mixture into a cooled (0-5° C) 20% sodium hydroxide aqueous solution and n-hexane or n-heptane. The product is obtained by filtration, and it is washed and dried. The pertinent results are given in Table 7. The products decompose at their melting points.

Table 7

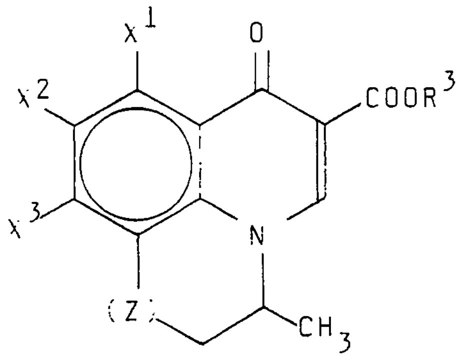

($X^1$=H)

| $X^2$ | $X^3$ | (Z) | $R^3$ | P.F.(°C) | C* | A* |
|---|---|---|---|---|---|---|
| F | $CH_3$ | CH | H | 268-272 | 100 | 92 |
| F | H | CH | H | 253-255 | 98 | 94 |
| F | $H_3CN(C_2H_4)N$ | O | H | 250-257 | 94 | 87 |
| F | $H_3CN(C_2H_4)N$ | S | H | --- | 98 | 91 |
| F | H | CH | $CH_3$ | 232-235 | 93 | 90 |
| $OCH_3$ | H | CH | H | 254-257 | 97 | 90 |
| F | H | CH | $C_2H_5$ | 176-178 | 88 | 80 |
| 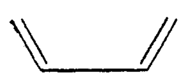 | | CH | H | --- | 100 | 92 |

* C = % conversion; A = % isolation.

Example 8

7-Chloro-6-fluoro-4-hydroxy-3-quinolinecarboxylic acid ethyl ester

Reagent SPPS (0.4 mole, N = 2) is prepared according to method of example 1A. Diethyl (4-fluoro-3-chloroanilino)methylenemalonate (31.6 g; 0.1 mole) is added, and the mixture is heated to 130°C during 15 min.. The solution thus obtained is cooled down to 70-80°C, and then methanol (400 ml) is graduately added during 30 min, in a cooling system. The resulting suspension is cooled further down to room temperature (20-25°C) and filtered. The solid is washed with a 1% sodium bicarbonate aqueous solution (400 ml) and with water (200 ml). After drying the title compound is obtained (24.21 g). Yield = 90%; m.p. 300°C.

Similar results are obtained when SPPS is replaced by SPPAS [$P_2O_5$: $(CH_3)_3SiO-CH_2CH_2-[OS_1(CH_3)_3]-CH_2OS_1(CH_3)_3$]. being N = 3.

Example 9

1-Cyclopropyl-6-fluoro-7-(1-piperazinyl)-1,4-dihydro-4-oxo-3-quinolinecarboxylic acid

To a suspension of phosphorus pentoxide (17.0 g; 12.0 cmole) in chloroform (24 ml) and HMDS (8.32 ml; 4.0 cmole), prepared as in example 1C (N = 3; D/SPPS = 2 ml/cmole), diethyl N-cyclopropyl-[3-(4-acetyl-1-piperazinyl)-4-fluoro]anilinomethylenemalonate (10.74 g; 2.4 cmole) (SPPS/P = 5). The stirred mixture is refluxed (about 70° C) for a time (60 min.; conversion > 98% , monitored by TLC). Water (75 ml) is added next and the chloroform distilled off. The heating is maintained for a short additional time (the hydrolytic separation of the acetyl and the carboxyacetyl is monitored by TLC). The pH is adjusted at 5-6 by adding a 20% sodium hydroxide aqueous solution. The precipitate formed, which is separated by filtration, washed successively with water, chloroform and methanol, is the title compound (7.5 g), m.p. = 255-258° C (dec). Yield = 94.5%.

Similar results are obtained when the SPPS is substituted by IP-SPPAS.

Example 10

1-cyclopropyl-6-fluoro-7-(4-ethyl-1-piperazinyl)-1,4-dihydro-4-oxo-3-quinolinecarboxylic acid

As in example 9, but substituting its aminomethylenemalonate by ditrimethylsilyl N-cyclopropyl-3-(4-ethyl-1-piperazinyl)anilinomethylenemalonate (11.84 g; 2.4 cmole). When the cyclization is over, dilution with water at room temperature is effected, and the pH is adjusted to 5-6. The precipitate formed, after being filtered, washed with water and dried, affords the title product (7.46 g), m.p. 219-221° C. The yield is 90.0%.

Similar results are achieved when the SPPS is substituted by M-SPPAS ($P_2O_5$: methoxytrimethylsilane).

Example 11

4-Hydroxy-7-methyl-1,8-naphthiridine-3-carboxylic acid

Carrying out the preparation of the reagent according to example 1A (N = 3; SPPS/P = 4), to a suspension of phosphorus pentoxide (56.8 g; 0.40 moles) in chlorobenzene (100 ml) hexamethyldisiloxane (27.8 ml; 0.133 mole) is added. Diethyl 6-methyl-2-pyridylaminomethylenemalonate (27.8 g; 0.1 mole) is added next, at 100° C and the resulting suspension is refluxed (132° C) for 20 min. About 5 min. later it becomes a yellowish solution which is cooled down to 70° C in a water bath, and a 10% sodium hydroxide aqueous solution (1000 ml) is gradually added (30 min.), keeping the temperature constant by adding ice onto the bath. The final pH is 7.5-8.0. The organic layer is decanted, and the aqueous layer is washed with dichloromethane (250 ml). The organic layer (chlorobenzene and dichloromethane), after drying over sodium sulfate and evaporated under vacuum, affords the ethyl ester of the title compound (20.9 g), m.p. 113-115° C, in a 90% yield. This ester is suspended in an aqueous solution of sodium hydroxide (150 ml; 10% w/v), being refluxed until it dissolves completely (60 min.). The reaction mass is cooled and, with acetic acid, adjusted to pH = 5. The precipitate formed is separated by filtration, washed with water and dried, giving the title compound (17.46 g), m.p. = 279-281° C, in a 95% yield.

Similar results are obtained when the SPPS is substituted by SPPAS ($P_2O_5$ : n-butyloxytrimethylsilane; N = 3.5), using diethyl (1-ethyl-6-methyl-2-pyridyl)aminomethylenemalonate.

Example 12

7,8-Benzo-4-hydroxy-3-quinolinecarboxylic acid

Preparing reagent SPPS (N= 2) by method A, example 1, to a suspension of phosphorus pentoxide (28.4 g; 0.20 mole) in chlorobenzene (50 ml) and hexamethyldisiloxane (21.4 ml; 0.10 mole), dietyl α-naphthylaminomethylenemalonate (16.45 g; 0.05 mole) is added. The stirred resulting mixture is heated to and kept at 130-132° C during 30 min.. The resulting mass, after cooling it down to room temperature (20-25° C), it is diluted with water (100 ml), and it is adjusted to pH = 5 with 30% (w/v) sodium hydroxide. The

precipitate formed is filtered, washed with water (100 ml) and dried, affording the ethyl ester of the title compound (12.74 g), m.p. = 262-263 °C. Yield = 90%.

The product is next suspended in a 10% (w/v) sodium hydroxide aqueous solution (50 ml), and heated to 100 °C for 120 min.. After cooling, more water (50 ml) is added and with acetic acid it is adjusted to pH = 5-6. The precipitate formed is separated by filtration, washed with water and dried giving the title compound (10.90 g), m.p. = 278-280 °C (dec.). Yield = 95%.

Similar results are reached when SPPS is substituted by SPPAS [$P_2O_5$ : $(CH_3)_3SiO-CH_2CH_2-OSi(CH_3)_3$].

Example 13

5-Methyl-9-fluoro-6,7-dihydro-1-oxo-1H,5H-benzo[ij]quinolizine-2-carboxylic acid

The reagent SPPS (20.0 cmole, N = 3) is prepared according to method of example 1C. Diethyl (2-methyl-6-fluorotetrahydro-quinolin-1-yl)methylenemalonate (13.37 g. 99%; 4.0 cmole) is added, and the mixture is heated to 70-75 °C for 60 min.. The resulting solution is cooled (20-25 °C) and next water is added (40 ml), the chloroform is distilled off, and it is heated at 100 °C during 150 min.. The precipitate formed is separated by filtration, washed with water (40 ml) and dried, giving the title compound (9.40 g). m.p. = 253-255 °C. Yield = 90%.

In the same manner, the tetrahydroquinoline is substituted by the product of the reaction between 5-carboxyamido-6-fluoro-tetrahydroquinaldine and diethyl trimethylsilyloxymethylene-malonate (reagent SPPS, N = 3 and SPPS/P = 5). Ethyl 8-cyano-6,7-dihydro-9-fluoro-5-methyl-1-oxo-1H,5H-benzo[ij]quinolizine-2-carboxylate, m.p. = 260-263 °C, results in a similar yield. Hydrolysis with refluxing a 1:1 mixture acetic acid/10% aqueous hydrochloric acid (60 min.) yields the carboxylic acid. m.p.> 300 °C (dec.).

Similar results are attained substituting SPPS by SPPAS [$P_2O_5$ : $(CH_3)_3SiO-CH_2CH_2OCH_3$].

Example 14

7,8-Benzo-1-ethyl-4-oxo-3-quinolinecarboxylic acid

To reagent SPPS (30.0 cmole), prepared according to method of example 1C, substituting the chloroform by dichloromethane (50.0 ml; $P_2O_5$ = 30.0 cmole; N = 3), diethyl (N-ethylanilino)-methylenemalonate (14.55 g: 5.0 cmole) at 25 °C is added. The reacting mixture is stirred at this temperature during 22 hours. Water (150 ml) is added next keeping the temperature at 25-30 °C with an ice/water bath: the dichloromethane is distilled off, and next at 25 °C, 220 ml of a 20% (w/v) sodium hydroxide aqueous solution is added, and the mixture is refluxed during 30 min. (100 °C). At 90-95 °C, by addition of acetic acid (20 ml, 35 cmole), a precipitate is formed which, after cooling to 25 °C, is filtered and washed with water. The dry product is the title compound (9.44 g), m.p. 256-258 °C. Yield = 87.0%. At the end of the reaction of 90% conversion was evaluated, which represents a selectivity of 99% and a yield of 96.7% referred to the evaluated conversion.

Similar results are reached subtituting SPPS by an SPPAS of example 4B.

Example 15

7-[3-(aminomethyl)-3-methyl-1-pyrrolidinyl]-1-cyclopropyl-6,8-difluoro-4-oxo-1,4-dihydro-3-quinolinecarboxylic acid

Diethyl N-cyclopropyl-2,4-difluoro[3(acetylaminoethyl)-3-methyl-1-pyrrolidinyl]anilinomethylenemalonate (4.53 g; 1.0 cmole) is added to reagent SPPS (5.0 cmole; N = 3) prepared in dichloromethane (10 ml), and then diluted with acetonitrile (5 ml). The mixture is stirred at 25-30 °C for a time (25 h.). Water (30 ml) is added, and the dichloromethane is distilled off. Next, a 20% (w/v) sodium hydroxide aqueous solution is added until an alkaline pH is reached, and then refluxed for a while (30 min.). While warm, acetic acid is

added to pH = 5-6, and a precipitate is formed which is filtered and washed with water, giving the title compound (3.33 g). Yield = 88.5%. This product is added to a solution of p-toluenesulfonic acid in 1,4-dioxane around 40° C. After stirring it for a while (30 min.), the mass is cooled, and the precipitate is filtered and washed with 1,4-dioxane, the tosylate, m.p.> 300° C, being obtained in a virtually quantitative yield. It shows in the I.R. the characteristic absorption between 1100-1200 $cm^{-1}$ due to the sulfonic group.

Example 16

7-[4-Succinyl-1-piperazinyl]-1-(2,4-difluorophenyl)-6-fluoro-1,4-dihydro-4-oxo-1,8-naphtharidine-3-carboxylic acid

In a first stage, N-piperazinylaspartic anhydride is made to react with 6-chloro-2-(2,4-difluorophenyl)-amino-5-fluoro-pyridine, in acetonitrile, at reflux temperature, in the presence of triethylamine, the triethylamine hydrochloride formed being filtered off. In a second stage, the concentrated solution of the resulting amine, is made to react with diethyl formylmalonate, with dimethyl trimethylsilyloxymethylenemalonate, or with diethyl chloromethylenemalonate, giving either AMME or AMMM. The yields of these two stages are 92.0 and 95.0, respectively. The product -no isolation is required- is added to reagent SPPS, N = 4, SPPS/P = 4, prepared in acetonitrile. The reaction mixture is stirred (20 h.) at 30° C. Next it is diluted with water, and the hydrolysis is performed (80° C). The pH is then adjusted first with sodium hydroxide and then with acetic acid, and the title compound, m.p. > 300° C (dec.), precipitates. The yield is 85.5%.

## Claims

1. A new organosilylpolyphosphoric reagent, its preparation and application to the synthesis of 3-carboxy-quinolones, azaquinolones and their salts, characterized by a composition given by formula I,

$[P_2O_5]_N \cdot [Y[(R^0)_3Si]R^1]$, (I)

where N is greater than 1 and smaller than 10; Y, an oxygen atom, two atoms of chlorine or bromine, or one of each; $R^0$, a linear or branched alkyl C1-C8; $R^1$, a linear or branched alkyl, methoxyethyl, ethoxyethyl, poly(ethyleneglycol ether), trialkylsilyloxyethyl, bis-2,3-trialkylsilyloxypropyl or 2-trialkylsilyloxypropyl, the alkyls being C1-C8, or a $(R^0)_3Si$ - where $R^0$ has the meaning given before- is prepared by the reaction of phosphorus pentoxide with trialkylchlorosilane, hexaalkyldisiloxane, alkyloxytrialkylsilane, 1,2-bistrialkylsilyloxyethane, 1,3-bistrialkylsilyloxypropane, 1,2-bistrialkylsilyloxypropane, 1,2,3-tristrialkylsilyloxypropane, 1-alkyloxy-2-trialkylsilyloxyethane, 1-methoxy-2,3-bistrialkylsilyloxypropane or a trialkylsilyloxypolyethyleneglycol ether, where the alkyls are C1-C8, and it is made to react, in an inert organic solvent, with an aminomethylenemalonate of formula II,

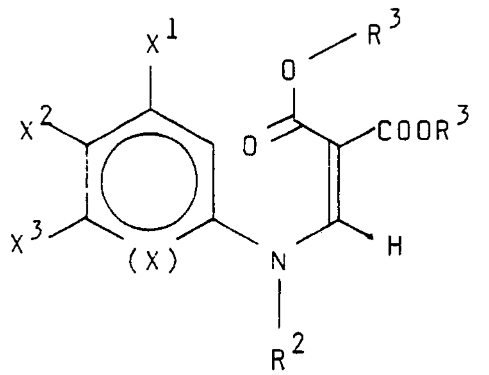

(II)

where (X) may be one N, C-F, C-H, $C-NO_2$, C-OH, C-COOH, $C-COOCH_3$; $X^1$, $X^2$, $X^3$ are chosen among hydro gen, fluorine, chlorine, bromine, alkyl C1-C4, nitro, sulfonic, carboxy, carboxyalkyl C1-C5, hydroxyl, methoxyl, methylenedioxyl, amino-dialkyl C1-C4, 1-piperazinyl, 4-alkyl(C1-C4)-1-piperazinyl, 4-acyl-1-piperazinyl with acyl C1-C8, di(β-hydroxyethyl)amino, di(β-chloroethyl)amino, aryl, substituted aryl, benzyl substi-tuted benzyl, or two of them bonded to give an alicyclic, aromatic, heterocyclic or with condensed

rings; $R^2$, a substituent chosen among hydrogen, hydrozyl, trialkylsilyl or alkyl C1-C4, cycloakyl C3-C7, cyclopropylmethyl, aryl, substituted aryl, benzyl, aminodialkyl C1-C4, or bonded to (X) when this is a carbon atom, and with which it forms a cycle of heterocycle, preferentially of six links, such as oxazine, thiazine, piperazine, piperidine or hydropyridine; and $R^3$, a linear alkyl C1-C5, isopropyl, terbutyl, benzyl, trimethylsilyl or a hydrogen atom, to obtain a quinolone or azaquinolone of formula III,

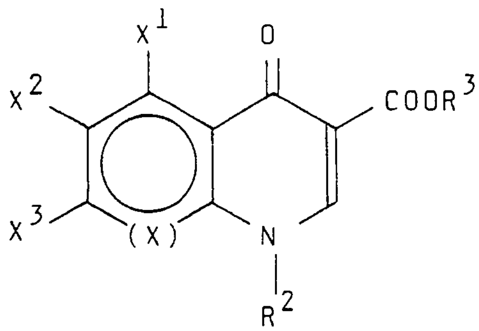

(III)

where (X), $X^1$, $X^2$, $X^3$, $R^2$ and $R^3$ have the meaning given above.

2. Process, according to claim 1, the characteristic of which is that phosphorus pentoxide is made to react with a trialkylsilyl polyphosphate, such as trimethylsilyl polyphosphate, an isopropyltrimethylsilyl polyphosphate, methyltrimethylsilyl, butyltrimethylsilyl, terbutyltrimethylsilyl, trimethylsilyloxypolyethyleneglycol ether, 1,2-bistrimethylsilyloxyethane, 1,3-bistrimethylsilyloxypropane, 1,2-bistrimethylsilyloxypropane, 1,2,3-tristrimethylsilyloxypropane, 1-methyloxy-2-trimethylsilyl oxyethane, 1-ethoxy-2-trimethylsilyloxyethane or 1-methoxy-2,3-bistrimethylsilyloxypropane, to obtain a compound of formula I.

3. Process, according to claim 1, the characteristic of which is that a compound of formula I is prepared, in a solvent chosen among benzene, toluene, xylenes, chlorobenzenes, dichloromethane, chloroform, trichloroethylene, polychloroethanes, polychloroethylenes, 1,4-dioxane, nitrobenzene or diphenylether, by heating preferentially between 20 and 135° C, a mixture of phosphorus pentoxide, in the molar proportion given by the values of N, and a compound chosen among a trialkylchlorosilane, hexaalkyldisiloxane, alkylsilyloxyalkylenoxytrimethylsilane, trialkylsilyloxyalkylenoxyalkane or an alkyloxytrimethylsilane which is made to react with an aminomethylenemalonate of formula II, during 5 min. to 24 h., preferentially at temperatures between 20° and 135° C, to obtain a mixture containing a compound of formula III, where $R^3$ is:

a) a hydrogen atom of a trimethylsilyl group. Water is added, adjusting the pH between 4 and 7. The quinolone or azaquinolone carboxylic acid of formula III is obtained.

b) a methyl or an ethyl. Water is added to perform, between 40° and 100° C, either the acid hydrolysis or sodium hydroxide for the basis hydolysis, finally adjusting to pH = 4-7 to obtain a quinolone or azaquinolone carboxylic acid of formula III.

c) a terbutyl or benzyl. It is meade to react with an oxonium salt, such as 1,4-dioxane hydrochloride, to obtain the hydrochloride of compound of formula III.

d) a trimethylsilyl. A methanol solution containing a sulfonic acid, sulfuric acid, phosphoric acid, hydrogen chloride or a carboxylic acid is added to obtain a pharmaceutically acceptable salt.

4. Process, according to claim 1, the characteristic of which is that a compound of formula I, for values of N greater than 1 and smaller than 8, preferentially from 3 to 6, is made to react with an aminomethylenemalonate of formula II to obtain a compound of formula III, the ratio I/II varying from 1 to 5.

5. Process, according to claim 1, for the preparation of an aminomethylenemalonate of formula II, the characteristic of which is that a compound of formula IV,

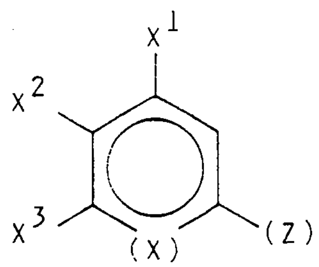

(IV)

where (X), $X^1$, $X^2$, and $X^3$ have the assignments given above, is made to react with a methylenemalonate of formula V,

$Z^1\text{-}CH = C(COOR^3)_2$ (V)

where $Z^1$ is a $N(R^2)H$ group, $R^2$ and $R^3$ have the meaning given above; Z is an atom of chlorine or a bromine; and $Z^1$ is chosen among hydroxyl, methoxyl, ethoxyl, trimethylsilyloxyl, chlorine, p-toluenesulfonate or methanesulfonate when Z is a $N(R^2)N$ group, being $R^2$ as above, which is made to react with a compound of formula I to give a carboxy quinolone or azaquinolone of formula III.

6. Process, according to claim 1, the characteristic of which is that a compound of formula I is made to react with a compound of formula II resulting from the reaction between an amine and a diethyl or bistrimethylsilyl trimethylsilyloxymethylenemalonate prepared "in situ", which in turn result from the reaction of the formylmalonate or the formylmalonic acid with a silylating agent chosen among trimethylchlorosilane, trimethylsilyl triflate, 3-trimethylsilyl-2-oxazolidinone or hexamethyldisilazane, in a solvent chosen among dichloromethane, chloroform, 1,2-dichloroethane or carbon tetrachloride, to obtain a quinolone of formula III.

7. A product of formula I, according to claim 1, the composition of which is

$[P_2O_5]_N \cdot [Cl\text{-}Si(CH_3)_3]_2$

where N has the assignment given above, characterized by its preparation which is effected by the reaction of trimethylchlorosilane with phosphorus pentoxide.

8. A product of formula I, according to claim 1, selected preferentially among those possessing the following compositions:

$[P_2O_5]_N \cdot [[(CH_3)_2CH](CH_3)_2Si\text{-}O\text{-}Si(CH_3)_2[CH(CH_3)_2]]$,
$[P_2O_5]_N \cdot [(H_3C)_3Si\text{-}O\text{-}Si(CH_3)_3]$,
$[P_2O_5]_N \cdot [(H_3C)_3Si\text{-}O\text{-}CH(CH_3)_2]$,
$[P_2O_5]_N \cdot [(H_3C)_3Si\text{-}O\text{-}C(CH_3)_3]$,
$[P_2O_5]_N \cdot [(H_3C)_3Si\text{-}O\text{-}CH_3]$,
$[P_2O_5]_N \cdot [(H_3C)_3Si\text{-}O\text{-}CH_2(CH_2)_2CH_3]$,

N having the meaning given above, characterized by its preparation which is effected by the reaction of the corresponding hexaalkyldisiloxane or an alkyloxytrime thylsilane with phosphorus pentoxide.

9. A product of formula I, according to claim 1, selected preferentially among those possessing the following compositions:

$[P_2O_5]_N \cdot [(H_3C)_3Si\text{-}O\text{-}CH_2\text{-}O\text{-}Si(CH_3)_3]$,
$[P_2O_5]_N \cdot [(H_3C)_3Si\text{-}O\text{-}CH_2CH_2\text{-}O\text{-}Si(CH_3)_3]$,
$[P_2O_5]_N \cdot [(H_3C)_3Si\text{-}O\text{-}CH_2CH_2\text{-}O\text{-}CH_3]$,
$[P_2O_5]_N \cdot [(H_3C)_3Si\text{-}O\text{-}CH_2CH[OSi(CH_3)_3]CH_2O\text{-}Si(CH_3)_3]$,
$[P_2O_5]_N \cdot [(H_3C)_3Si\text{-}O\text{-}CH_2CH[OSi(CH_3)_3]CH_3$,
$[P_2O_5]_N \cdot [(H_3C)_3Si\text{-}O\text{-}CH_2CH_2\text{-}O\text{-}CH_2CH_3]$,
$[P_2O_5]_N \cdot [(H_3C)_3Si\text{-}O\text{-}CH_2CH_2CH_2\text{-}O\text{-}Si(CH_3)_3]$,

N having the meaning given above, characterized by its preparation which is effected by the reaction of an alkylidenetrimethylsiloxane with phosphorus pentoxide.

10. A product of formula V according to claim 5, represented by formula VI,

$(CH_3)_3Si\text{-}O\text{-}CH = C(COOC_2H_5)_2$ (VI)

characterized by its preparation by the reaction of diethyl hydromethylenemalonate with a silylating agent chosen preferentially among trimethylsilylchlorosilane, hexamethyl-disilazane or 3-trimethylsilyl-2-oxazolidinone.

11. A product of formula V according to claim 5, represented by formula VII,

$(CH_3)_3Si\text{-}O\text{-}CH = C[COOSi(CH_3)_3]_2$ (VII)

characterized by its preparation by the reaction of hydroxymethylenemalonic acid or its alkaline or alkaline-earth salt with silylating agent, trimethylchlorosilane preferentially.

12. A product of formula V, according to claim 5, chosen preferentially among the compounds represented by the following formulae:

$C_2H_5$-NH-CH = C($COOC_2H_5$)$_2$

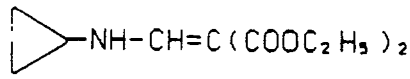

($H_3C$)$_2$CH-NH-CH = C($COOC_2H_5$)$_2$

($H_3C$)$_3$C-NH-CH = C($COOC_2H_5$)$_2$

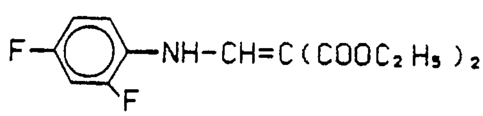

characterized by its preparation by the reaction of the corresponding amine with a compound of formula V, preferentially those of formulae VI, VII, a diethyl ethoxymethylenemalonate or a diethyl hydroxymethylenemalonate.

Claims for the following Contracting State: ES

1. A process for the preparation of a new organosilylpolyphosphoric reagent, and its application to the synthesis of 3-carboxy-quinolones, azaquinolones and their salts, characterized by a composition given by formula I,

$[P_2O_5]_N \cdot [Y[(R^0)_3Si]R^1]$, (I)

where N is greater than 1 and smaller than 10; Y, an oxygen atom, two atoms of chlorine or bromine, or one of each; $R^0$, a linear or branched alkyl C1-C8; $R^1$, a linear or branched alkyl, methoxyethyl, ethoxyethyl, poly(ethyleneglycol ether), trialkylsilyloxyethyl, bis-2,3-trialkylsilyloxypropyl or 2-trialkylsilyloxypropyl, the alkyls being C1-C8, or a $(R^0)_3$Si -where $R^0$ has the meaning given before- is prepared by the reaction of phosphorus pentoxide with trialkylchlorosilane, hexaalkyldisiloxane, alkyloxytrialkylsilane, 1,2-bistrialkylsilyloxyethane, 1,3-bistrialkylsilyloxypropane, 1,2-bistrialkylsilyloxypropane, 1,2,3-tristrialkylsilyloxypropane, 1-alkyloxy-2-trialkylsilyloxyethane, 1-methoxy-2,3-bistrialkylsilyloxypropane or a trialkylsilyloxypolyethyleneglycol ether, where the alkyls are C1-C8, and it is made to react, in an inert organic solvent, with an aminomethylenemalonate of formula II,

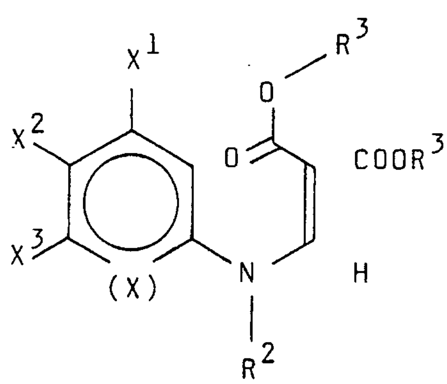

(II)

where (X) may be one N, C-F, C-H, C-$NO_2$, C-OH, C-COOH, C-$COOCH_3$; $X^1$, $X^2$, $X^3$ are chosen among hydrogen, fluorine, chlorine, bromine, alkyl C1-C4, nitro, sulfonic, carboxyl, carboxyalkyl C1-C5, hydroxyl, methoxyl, methylenedioxyl, amino-dialkyl C1-C4, 1-piperazinyl, 4-alkyl(C1-C4)-1-piperazinyl, 4-acyl-1-piperazinyl with acyl C1-C8, di($\beta$-hydroxyethyl)amino, di($\beta$-chloroethyl)amino, aryl, substituted aryl, benzyl substi-tuted benzyl, or two of them bonded to give an alicyclic, aromatic, heterocyclic or with condensed rings; $R^2$, a substituent chosen among hydrogen, hydroxyl, trialkylsilyl or alkyl C1-C4, cycloalkyl C3-C7, cyclopropylmethyl, aryl, susbtituted aryl, benzyl, aminodialkyl C1-C4, or bonded to (X) when this is a carbon

atom, and with which it forms a cycle or heterocycle, preferentially of six links, such as oxazine, thiazine, piperazine, piperidine or hydropyridine; and $R^3$, a linear alkyl C1-C5, isopropyl, terbutyl, benzyl, trimethylsilyl or a hydrogen atom, to obtain a quinolone or azaquinolone of formula III,

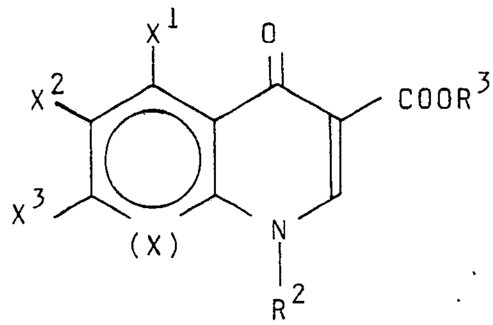

(III)

where (X), $X^1$, $X^2$, $X^3$, $R^2$ and $R^3$ have the meaning given above.

2. Process, according to claim 1, the characteristic of which is that phosphorus pentoxide is made to react with a trialkylsilyl polyphosphate, such as trimethylsilyl polyphosphate, an isopropyltrimethylsilyl polyphosphate, methyltrimethylsilyl, butyltrimethylsilyl, terbutyltrimethylsilyl, trimethylsilyloxypolyethyleneglycol ether, 1,2-bistrimethylsilyloxyethane, 1,3-bistrimethylsilyloxypropane, 1,2-bistrimethylsilyl oxypropane, 1,2,3-tristrimethylsilyloxypropane, 1-methyloxy-2-trimethylsilyl-oxyethane, 1-ethoxy-2-trimethylsilyloxyethane or 1-methoxy-2,3-bistrimethylsilyloxypropane, to obtain a compound of formula I.

3. Process, according to claim 1, the characteristic of which is that a compound of formula I is prepared, in a solvent chosen among benzene, toluene, xylenes, chlorobenzenes, dichloromethane, chloroform, trichloroethylene, polychloroethanes, polychloroethylenes, 1,4-dioxane, nitrobenzene or diphenylether, by heating preferentially between 20 and 135° C, a mixture of phosphorus pentoxide, in the molar proportion given by the values of N, and a compound chosen among a trialkylchlorosilane, hexaalkyldisiloxane, alkylsilyloxyalkylenoxytrimethylsilane, trialkylsilyloxyalkylenoxyalkane or an alkyloxytrimethylsilane which is made to react with an aminomethylenemalonate of formula II, during 5 min. to 24 h., preferentially at temperatures between 20° and 135° C, to obtain a mixture containing a compound of formula III, where $R^3$ is:

a) a hydrogen atom of a trimethylsilyl group. Water is added, adjusting the pH between 4 and 7. The quinolone or azaquinolone carboxylic acid of formula III is obtained.

b) a methyl or an ethyl. Water is added to perform, between 40° and 100° C, either the acid hydrolysis or sodium hydroxide for the basic hydrolysis, finally adjusting to pH = 4-7 to obtain a quinolone or azaquinolone carboxylic acid of formula III.

c) a terbutyl or benzyl. It is meade to react with an oxonium salt, such as 1,4-dioxane hydrochloride, to obtain the hydrochloride of compound of formula III.

d) a trimethylsilyl. A methanol solution containing a sulfonic acid, sulfuric acid, phosphoric acid, hydrogen chloride or a carboxylic acid is added to obtain a pharmaceutically acceptable salt.

4. Process, according to claim 1, the characteristic of which is that a compound of formula I, for values of N greater than 1 and smaller than 8, preferentially from 3 to 6, is made to react with an aminomethylenemalonate of formula II to obtain a compound of formula III, the ratio I/II varying from 1 to 5.

5. Process, according to claim 1, for the preparation of an aminomethylenemalonate of formula II, the characteristic of which is that a compound of formula IV.

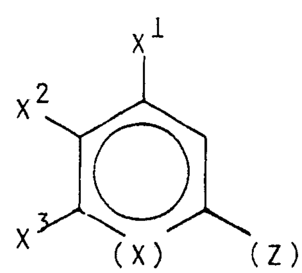

(IV)

where (X), $X^1$, $X^2$, and $X^3$ have the assignments given above, is made to react with a methylenemalonate of

formula V,

$Z^1\text{-}CH=C(COOR^3)_2$ (V)

where $Z^1$ is a $N(R^2)H$ group, $R^2$ and $R^3$ have the meaning given above; Z is an atom of chlorine or a bromine; and $Z^1$ is chosen among hydroxyl, methoxyl, ethoxyl, trimethylsilyloxyl, chlorine, p-toluenesulfonate or methanesulfonate when Z is a $N(R^2)N$ group, being $R^2$ as above, which is made to react with a compound of formula I to give a carboxy quinolone or azaquinolone of formula III.

6. Process, according to claim 1, the characteristic of which is that a compound of formula I is made to react with a compound of formula II resulting from the reaction between an amine and a diethyl or bistrimethylsilyl trimethylsilyloxymethylenemalonate prepared "in situ", which in turn result from the reaction of the formylmalonate of the formylmalonic acid with a silylating agent chosen among trimethylchlorosilane, trimethylsilyl triflate, 3-trimethylsilyl-2-oxazolidinone or hexamethyldisilazane, in a solvent chosen among dichloromethane, chloroform, 1,2-dichloroethane or carbon tetrachloride, to obtain a quinolone of formula III.

7. Process, according to claim 1, for the preparation of a product of formula I, the composition of which is

$[P_2O_5]_N \cdot [Cl\text{-}Si(CH_3)_3]_2$

where N has the assignment given above, characterized by its preparation which is effected by the reaction of trimethylchlorosilane with phosphorus pentoxide.

8. Process, according to claim 1, for the preparation of a compound of formula I selected preferentially among those possessing the following compositions;

$[P_2O_5]_N \cdot [[(CH_3)_2CH](CH_3)_2Si\text{-}O\text{-}Si(CH_3)_2[CH(CH_3)_2]]$,

$[P_2O_5]_N \cdot [(H_3C)_3Si\text{-}O\text{-}Si(CH_3)_3]$,

$[P_2O_5]_N \cdot [(H_3C)_3Si\text{-}O\text{-}CH(CH_3)_2]$,

$[P_2O_5]_N \cdot [(H_3C)_3Si\text{-}O\text{-}C(CH_3)_3]$,

$[P_2O_5]_N \cdot [(H_3C)_3Si\text{-}O\text{-}CH_3]$,

$[P_2O_5]_N \cdot [(H_3C)_3Si\text{-}O\text{-}CH_2(CH_2)_2CH_3]$,

N having the meaning given above, characterized by its preparation which is effected by the reaction of the corresponding hexaalkyldisiloxane or an alkyloxytrimethylsilane with phosphorus pentoxide.

9. Process, according to claim 1, for the preparation of a compound of formula I selected preferentially among those possessing the following compositions:

$[P_2O_5]_N \cdot [(H_3C)_3Si\text{-}O\text{-}CH_2\text{-}O\text{-}Si(CH_3)_3]$,

$[P_2O_5]_N \cdot [(H_3C)_3Si\text{-}O\text{-}CH_2CH_2\text{-}O\text{-}Si(CH_3)_3]$,

$[P_2O_5]_N \cdot [(H_3C)_3Si\text{-}O\text{-}CH_2CH_2\text{-}O\text{-}CH_3]$,

$[P_2O_5]_N \cdot [(H_3C)_3Si\text{-}O\text{-}CH_2CH[OSi(CH_3)_3]CH_2O\text{-}Si(CH_3)_3]$,

$[P_2O_5]_N \cdot [(H_3C)_3Si\text{-}O\text{-}CH_2CH[OSi(CH_3)_3CH_3$,

$[P_2O_5]_N \cdot [(H_3C)_3Si\text{-}O\text{-}CH_2CH_2\text{-}O\text{-}CH_2CH_3]$,

$[P_2O_5]_N \cdot [(H_3C)_3Si\text{-}O\text{-}CH_2CH_2CH_2\text{-}O\text{-}Si(CH_3)_3]$,

N having the meaning given above, characterized by its preparation which is effected by the reaction of the corresponding alkylidenetrimethylsiloxane with phosphorus pentoxide.

10. Process, according to claim 5, for the preparation of a compound of formula V represented by formula VI,

$(CH_3)_3Si\text{-}O\text{-}CH=C(COOC_2H_5)_2$ (VI)

characterized by its preparation by the reaction of diethyl hydromethylenemalonate with a silylating agent chosen preferentially among trimethylsilylchlorosilane, hexamethyl-disilazane or 3-trimethylsilyl-2-oxazolidinone.

11. Process, according to claim 5, for the preparation of a compound of formula V represented by formula VII,

$(CH_3)_3Si\text{-}O\text{-}CH=C[COOSi(CH_3)_3]_2$ (VII)

characterized by its preparation by the reaction of hydroxymethylenemalonic acid or its alkaline or alkaline-earth salt with a silylating agent, trimethylchlorosilane preferentially.

12. Process, according to claim 5, for the preparation of a compound of formula V chosen preferentially among the compounds represented by the following formulae:

$C_2H_5\text{-}NH\text{-}CH=C(COOC_2H_5)_2$

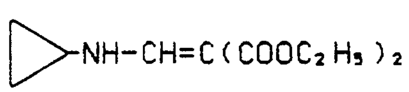

$(H_3C)_2CH\text{-}NH\text{-}CH = C(COOC_2H_5)_2$
$(H_3C)_3C\text{-}NH\text{-}CH = C(COOC_2H_5)_2$

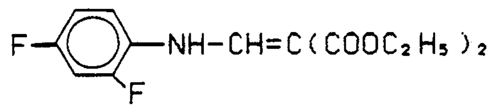

characterized by its preparation by the reaction of the corresponding amine with a compound of formula V, selecting preferentially those of formulae VI, VII, a diethyl ethoxymethylenemalonate or a diethyl hydroxymethylenemalonate.

European Patent Office

# EUROPEAN SEARCH REPORT

Application Number

EP 89 50 0046

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| D,X | TETRAHEDRON LETTERS, vol. 22, no. 19, 1981, pages 1803-1804, Pergamon Press, Oxford, GB; T. IMAMOTO et al.: "Trimethylsilyl polyphosphate (PPSE)- A useful reagent for the Beckmann rearrangement" * Whole document * | 1,8 | C 07 F 9/09<br>C 07 D 215/56<br>C 07 D 471/04<br>C 07 D 498/06<br>C 07 F 7/18<br>C 07 C 229/30 |
| D,X | CHEMISTRY LETTERS, no. 8, August 1982, pages 1225-1228, The Chemical Society of Japan; K. YAMAMOTO et al.: "Composition of "polyphosphoric acid trimethylsilyl ester (PPSE)" and its use as a condensation reagent" * Whole document * | 1,8 | |
| X | SYNTHESIS, no. 7, July 1982, pages 591-592, Georg Thieme Verlag, Stuttgart, DE; M. YOKOYAMA et al.: "Organic reactions using trimethylsilyl polyphosphate (PPSE): A convenient synthesis of nitriles from carboxamides" * Whole document * | 1,8 | **TECHNICAL FIELDS SEARCHED (Int. Cl.5)** |
| X | US-A-3 639 440 (K.D. KAMPE) * Column 2, lines 32-49; examples 16-20; claims * | 1,8 | C 07 F 9/00<br>C 07 D 215/00<br>C 07 D 471/00<br>C 07 D 498/00 |
| A | GB-A-1 324 331 (SUMITOMO CHEMICAL CO.) * Whole document * | 1-12 | |

The present search report has been drawn up for all claims

| Place of search | Date of completion of the search | Examiner |
|---|---|---|
| THE HAGUE | 20-02-1990 | BESLIER L.M. |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)